# FASCICULE DE BREVET EUROPEEN

(11) **EP 1 740 269 B1**
(45) Date de publication et mention de la délivrance du brevet: **31.07.2013**
(21) Numéro de dépôt: 05729795.4
(22) Date de dépôt: 30.03.2005
(51) Int. Cl.: A61N 5/06

(54) **DISPOSITIF DE PHOTOTHERAPIE**
PHOTOTHERAPEUTISCHE VORRICHTUNG
PHOTOTHERAPY DEVICE

(30) Priorité: 31.03.2004 BE 200400167
(43) Date de publication de la demande: 10.01.2007
(73) Titulaire: Constructions Electriques Schreder, 4430 Ans (BE)
(72) Inventeur: POIRRIER, Robert, B-4052 Beaufays (BE); MOREAU, Vincent, B-4910 Theux (BE)
(74) Mandataire: Luys, Marie-José A.H.
(86) Numéro de dépôt international: PCT/EP2005/051430
(87) Numéro de publication internationale: WO 2005/094941

(56) Documents cités:
- EP-A- 0 302 035
- WO-A-89/08476
- WO-A-90/10473
- WO-A-91/14475
- WO-A-94/09851
- WO-A-02/074176
- WO-A-2004/096364
- US-A- 5 923 398
- US-A- 6 053 936
- US-B1- 6 235 046

## Description

La présente invention concerne un dispositif photothérapie, actif au niveau des yeux d'un individu par des rayons lumineux d'au moins une longueur d'onde déterminée, les rayons étant émis par au moins une source lumineuse fixe par rapport à sa tête.

La photothérapie appliquée à l'homme semble être un traitement efficace, notamment contre le Trouble Affectif Saisonnier qui est une forme de dépression dont la principale caractéristique est de survenir durant la même période, chaque année, habituellement au début de l'automne ou de l'hiver pour se terminer au printemps.

Appliquée à un individu, la photothérapie (appelée parfois luminothérapie), peut consister à l'exposer à un éclairement lumineux important (entre 2500 et 10000 lux sur la cornée) pendant une certaine période dans un environnement contrôlé. L'information relative à cet éclairement, captée par les yeux, est alors transmise via le nerf optique au noyau suprachiasmatique de l'hypothalamus qui régule les rythmes circadiens neurologiques est endocriniens, avec un impact sur la vigilance, l'humeur, les performances physiques et psychologiques de l'individu.

La mélatonine est un des marqueurs privilégiés des effets de la lumière sur le noyau suprachiasmatique et l'épiphyse.

Des paramètres photométriques importants pour un traitement efficace sont donc
- le flux lumineux proportionnel à la quantité de photons reçus par l'oeil humain par unité de temps, exprimé soit en lumen, soit en watt,
- l'éclairement incident, défini comme le flux lumineux par unité de surface et exprimé soit en lux, soit en watt/m²,
- la quantité d'éclairement, définie par le produit de l'éclairement par la durée d'exposition, qui s'exprime en lux.s.

Des dispositifs de photothérapie sont principalement connus sous deux formes, à savoir :
- des appareils d'éclairement non portables qui obligent l'individu en traitement à rester en place pendant chaque séance d'exposition et qui, si l'individu souhaite par exemple lire pendant ce temps, perdent considérablement de leur efficacité en raison de ce que l'individu baisse les paupières qui forment alors un écran à l'éclairement de traitement, et
- des appareils d'éclairement portables fixés à la tête, à la manière d'un casque ou d'un serre-tête ou sous forme de lunettes, qui soit éclairent les yeux directement depuis le haut et souffrent du même problème des paupières qui font obstacle à l'éclairement visé lorsque l'individu veut notamment lire, soit ne permettent aucune activité parce qu'elles inondent de lumière tout le champ visuel. Le document US-A-6235046 décrit un appareil selon le préambule de la revendication 1.

La présente invention a pour but de remédier aux inconvénients des dispositifs connus et vise à cet effet les objectifs suivants :
- la source lumineuses et son alimentation doivent être légères et peu encombrantes. Elles sont associées à un boîtier de commande permettant de régler le flux émis par la source et la durée de fonctionnement. Ce boîtier est séparé du moyen d'éclairement thérapeutique et est portable (par exemple à la ceinture),
- le moyen d'éclairement des yeux doit être léger et discret. Afin de réduire les pertes de puissance lumineuse, ce système est situé aussi près que possible de l'oeil de l'utilisateur. Une solution élégante consiste à lui donner la forme d'une paire de lunettes non correctrices, adaptable sur une paire de lunettes correctrices et pouvant fournir une quantité d'éclairement thérapeutique efficace,
- le moyen d'éclairement demeure suffisamment transparent afin de permettre au patient de pratiquer diverses activités semi-sédentaires durant le traitement (lecture, travail ou loisir sur écran, déplacements sécurisés...),
- le spectre lumineux délivré par le moyen d'éclairement doit contenir une ou des longueurs d'onde choisies, ou toutes, de la lumière visible tout en garantissant l'exclusion des rayonnements nocifs ultraviolets et infrarouges. Ce spectre est à adapter en fonction des résultats d'études en vue d'optimiser le traitement par photothérapie,
- enfin, le coût de fabrication de l'ensemble du dispositif doit être suffisamment faible pour permettre de le proposer à un prix avantageux par rapport à celui des dispositifs connus, fixes ou portables.

A cet effet, la présente invention propose un dispositif dans lequel la source lumineuse est disposée à la périphérie du champ de vision permettant les activités usuelles de l'individu et qui dévie les rayons lumineux susdits sur une zone déterminée de la rétine, de manière à conserver une vision.

Cette disposition est bien sûr avantageusement appliquée aux deux yeux de l'individu à traiter, si ceux-ci sont à même de recevoir utilement cette lumière.

Dans un mode de réalisation de l'invention, on choisit la zone susdite recevant les rayons déviés de manière à exclure la fovéa quelle que soit la direction du regard en dessous d'un plan passant par les axes optiques de lentilles agencées pour dévier les rayons lumineux vers ladite zone déterminée. Une activité demeure ainsi possible, la fovéa étant la zone la plus sensible de l'oeil permettant une vision fine et l'analyse de détails dans les activités semi-sédentaires précitées.

Avantageusement, on fait converger dans l'oeil, les rayons lumineux déviés, en un lieu situé légèrement en arrière de la pupille de l'oeil. Cette convergence des rayons entrant dans le globe oculaire permet d'éclairer une surface étendue sur la rétine, même lorsque l'oeil change de direction de regard dans les limites précitées, et donc accroît sensiblement l'effet bénéfique de ces rayons.

On dévie les rayons lumineux par diffraction.

Suivant l'invention, le dispositif comprend un support destiné à être immobilisé sur la tête ainsi que la ou les sources lumineuses montées sur le support à la périphérie du champ de vision, émettant des rayons lumineux d'au moins une longueur d'onde déterminée, et agencées pour que ces rayons soient dirigés dans les yeux, par des moyens de déviation, sur la zone déterminée précitée. Bien que la lumière blanche, sans UV ni infrarouge puisse convenir, une sélection du bleu semble avoir un intérêt certain. Entre autres, une irisation donnée par la lumière blanche, par diffraction, disparaît avec une sélection d'une couleur.

Dans une forme de réalisation de l'invention, le support précité est constitué par une monture de lunettes, les moyens de déviation susdits se présentant sous la forme de verres de lunettes.

Ledit dispositif comporte pour chaque oeil une ou plusieurs sources lumineuses, telles que diodes électroluminescentes, et des moyens de déviation distincts agencés pour coopérer avec la ou les sources lumineuses de chaque oeil.

Avantageusement, il y a pour chaque source lumineuse un condenseur qui est agencé pour orienter les rayons lumineux émis par chacune des sources sur les moyens de déviation précités, et qui est associé à la source lumineuse à la périphérie du champ de vision.

Les moyens de déviation sont constitués par une lentille diffractive, constituée d'un élément optique diffractif hors axe, pour chaque oeil.

D'autres détails et particularités de l'invention ressortiront des revendications secondaires et de la description des dessins qui sont annexés au présent mémoire et qui illustrent, à titre d'exemples non limitatifs. Les formes de réalisation particulières du dispositif suivant l'invention.
La figure 1 montre schématiquement dans une vue en perspective, avec brisure, un dispositif de l'invention.
La figure 2 montre schématiquement, en coupe verticale, un cheminement de rayons lumineux depuis une source lumineuse du dispositif de l'invention jusqu'au fond de l'oeil d'un individu équipé de ce dispositif.

Dans les différentes figures, les mêmes notations de référence désignent des éléments identiques ou analogues.

Pour la facilité de l'explication et de la compréhension de l'invention, le dispositif de l'invention sera décrit, avant le procédé, dans le cadre de son application à un individu, sans que cela ne puisse en limiter une application à des animaux, ni le procédé de l'invention.

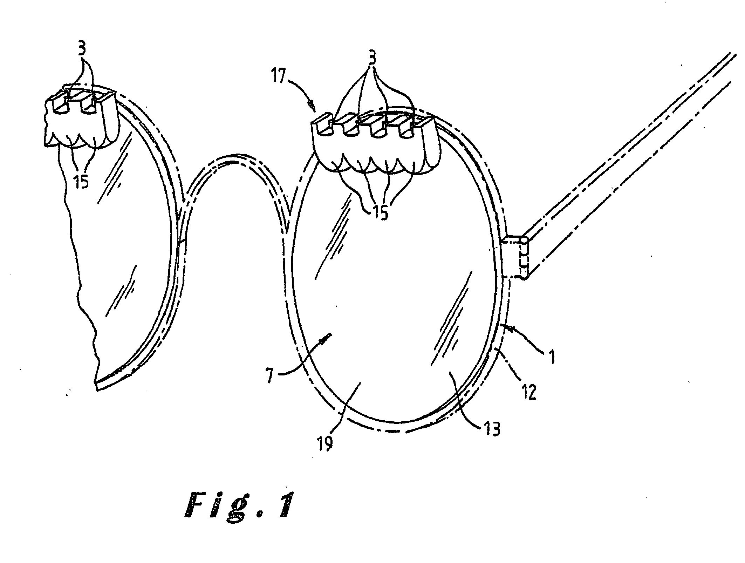

Un dispositif pour la mise en oeuvre du procédé de photothérapie suivant l'invention comprend (figure 1) un support 1 destiné à être immobilisé sur la tête (non représentée) d'un individu.

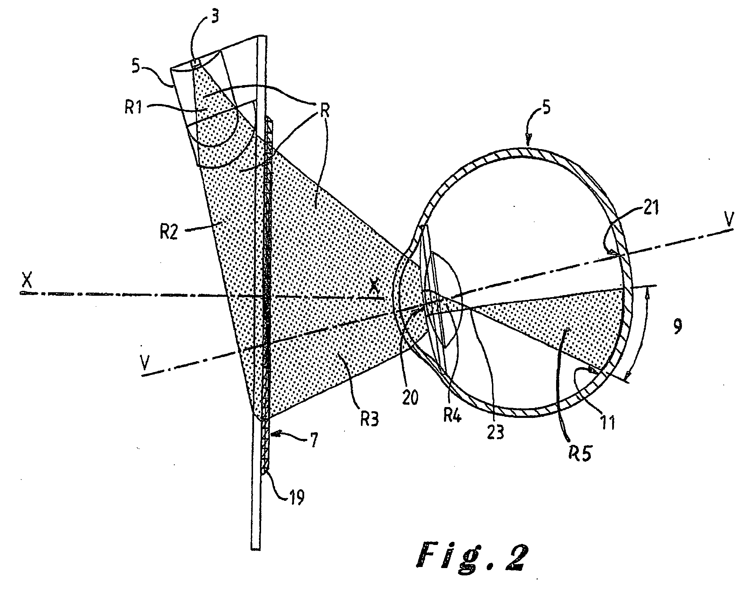

Une ou des sources lumineuses 3 sont montées sur le support 1 à la périphérie du champ de vision de l'individu. Les sources 3 émettent des rayons lumineux R (figure 2) d'au moins une longueur d'onde déterminée, et elles sont agencées pour que ces rayons R soient dirigés dans les yeux 5 de l'individu, par des moyens de déviation 7, sur une zone déterminée 9 de la rétine 11.

Le support 1 précité peut être constitué par une monture .de lunettes 12, les moyens de déviation 7 susdits se présentant alors sous la forme des verres 13 de ces lunettes.

A noter que dans la présente description, par verre il faut entendre tout objet transparent, correcteur ou non, agencé pour être monté dans une monture de lunettes ou autre support 1, devant des yeux 5, et permettant de voir au travers, quelle que soit sa matière, sa couleur, ....

Suivant une variante de l'invention, le support 1 peut être constitué, d'une part, d'une monture de lunettes 12 usuelle à verres correcteurs et, d'autre part, d'une applique de lunettes (non représentée) connue en soi notamment sous la forme d'un élément qui peut être équipé de verres solaires non correcteurs et qui est agencé pour être fixé de manière amovible sur des lunettes correctrices. Les moyens de déviation 7 susdits se présentent alors sous la forme des verres 13 de ladite applique et la ou les sources lumineuses 3 sont montées sur cette applique.

Dans une forme de réalisation non représentée de l'invention, les lunettes peuvent aussi être constituées à la manière de lunettes de protection de skieurs, et ne comporter qu'un verre ou écran d'une pièce devant les deux yeux, comme cela est connu en soi. La ou les sources lumineuses 3 peuvent alors être groupées par exemple au-dessus du nez de l'individu qui portera ces lunettes.

Le dispositif suivant l'invention comporte pour chaque oeil 5 :
- une ou plusieurs sources lumineuses 3, notamment quatre par oeil comme le montrent les dessins, celles-ci étant de préférence des diodes électroluminescentes pour des raisons d'encombrement, de poids et de consommation, et
- des moyens de déviation 7 distincts agencés pour coopérer avec la ou les sources lumineuses 3 de chaque oeil 5.

Des diodes électroluminescentes sont choisies parce qu'elles peuvent présenter les caractéristiques suivantes pour l'application développée :
- leur rendement énergétique (puissance émise/puissance consommée) est l'un des plus intéressants parmi les différents types de sources lumineuses disponibles,
- il est actuellement possible de trouver sur le marché des diodes de toutes couleurs, depuis l'ultraviolet jusqu'à l'infrarouge,
- les flux de lumière émis peuvent atteindre au moins une dizaine de lumens pour les plus puissantes.
- la chaleur dégagée par ces sources 3 est très faible ; elles peuvent donc être placées sans inconvénient sur le support de lunettes,
- la durée de vie des diodes électroluminescentes dépasse 100 000 heures,
- leur prix est peu élevé.

Les contraintes d'encombrement amènent à opter pour des diodes de type SMD (Surface Mounted Device = dispositif monté en surface).

Pour accroître l'efficacité ou rendement lumineux du dispositif de l'invention, celui-ci comporte avantageusement, de préférence distinct pour chaque source lumineuse 3, un condenseur 15 qui est agencé pour orienter les rayons lumineux R émis par chacune des sources 3 sur les moyens de déviation 7 précités, et qui est associé à ladite source lumineuse 3 à la périphérie du champ de vision de l'individu équipé du dispositif de l'invention. Des lentilles de Fresnel peuvent servir à construire un condenseur 15 à encombrement minimal et obtenir un flux de lumière à répartition optimale sur les moyens de déviation 7.

La figure 1 montre une forme de réalisation comprenant pour chaque oeil 5 un ensemble monobloc 17 qui regroupe chaque fois les quatre condenseurs 15 disposés entre les quatre diodes électroluminescentes et les moyens de déviation 7 correspondants. Du côté des diodes, cet ensemble monobloc 17 est creusé en face de chaque diode de façon à au moins en entourer partiellement la face émettrice de lumière et canaliser un maximum de rayons vers lesdits moyens de déviation 7.

Les moyens de déviation 7 ont constitués pour chaque oeil par une lentille diffractive 19, constituée d'un élément optique diffractif hors - axe". On connaît actuellement sous leur vocable anglais de DOE (Diffractive Optical Element) des éléments diffractifs dans lesquels, en traversant un milieu à microstructure, un front d'onde lumineuse incidente est fractionné en une multitude de petites ondes secondaires qui, en se recombinant vont former un front d'onde complètement nouveau. Le choix de la matière de ces éléments diffractifs est vaste : verre, matières synthétiques diverses, etc.

La réalisation d'une lentille diffractive hors - axe 19 présentant une ouverture numérique élevée peut mettre en oeuvre un procédé d'enregistrement holographique dans une résine photosensible (du type de celles utilisées en microélectronique). Le principe consiste à provoquer une interférence entre deux fronts d'ondes cohérents, l'un plan et l'autre sphérique. Cette interférence se traduit par une modulation, à l'échelle du micromètre, de l'éclairement tombant sur une couche mince de résine photosensible déposée sur un substrat de verre. Un développement chimique permet de dissoudre les parties ayant reçu une quantité d'éclairement élevée, produisant une modulation souhaitée de l'épaisseur de la couche de résine développée.

La production en masse de réseaux diffractifs en relief de surface est un problème qui est aujourd'hui bien maîtrisé par plusieurs fabricants. Le développement des supports optiques pour le stockage d'informations, et en particulier le compact disque et le DVD, ont largement contribué à ce savoir-faire. Les techniques de réplication actuelles atteignent une résolution de l'ordre du nanomètre et le coût d'une réplique est relativement indépendant de la complexité de la microstructure originale.

Si souhaité, des répliques du composant original peuvent être réalisées indépendamment dudit substrat de verre et présenter une face adhésive de façon à pouvoir être collée ultérieurement sur un verre 13 de lunettes par exemple correcteur avant d'installer sur les mêmes lunettes les sources lumineuses 3, condenseurs 15, etc.

Les lentilles diffractives 19 précitées peuvent être caractérisées selon plusieurs critères. Les caractéristiques géométriques (profondeur de gravure, pas du réseau, facteur de forme) ont une importance primordiale dans le choix des procédés de reproduction à grande échelle. Il faut noter que dans le cas d'une lentille diffractive précitée, contrairement a celui d'un réseau linéaire, ces paramètres ne sont pas constants sur toute la surface de la lentille. Les propriétés optiques, principalement le rendement de diffraction, permettent d'évaluer les pertes lumineuses du système.

Le tableau ci-après donne à titre d'exemple des valeurs pour les critères susdits :

| | |
|---|---|
| Période maximale | 1 µm |
| Période minimale | 0,4 µm |
| Profondeur de gravure | 0,4 µm |
| Facteur de forme (Profondeur/Période) | de 0,4 à 1 |
| Longueur focale | 35 mm @514,5 nm |
| Rendement de diffraction (Flux diffracté/Flux incident) | entre 30 % et 37% |

Si l'on observe la valeur mesurée du rendement de diffraction, on aperçoit qu'un tiers du flux lumineux qui frappe la lentille diffractive 19 est effectivement redirigé vers la pupille 20 du porteur de celle-ci. Cette valeur peut sembler faible mais elle est à comparer à une valeur théorique maximale calculée qui est de 41%. De plus, une valeur trop élevée du rendement de diffraction hypothéquerait la transparence relative du présent verre de lunettes ou lentille 19 et rendrait son utilisation peu confortable.

Le condenseur de rayons lumineux 15 est avantageusement agencé pour orienter lesdits rayons R sur la face de la lentille de diffraction 19 correspondante suivant un angle d'incidence rasante de l'ordre de 10° par rapport à l'axe optique X-X de cette lentille, la distance séparant cette dernière de l'oeil 3 étant telle que la l'image réelle 23 de la source lumineuse est située dans l'oeil 3, légèrement en arrière de la pupille 20 de celui-ci.

Une ouverture numérique de la lentille diffractive 19 de l'ordre de 0,7 s'avère être un choix favorable.

Un ajustement relativement précis de différentes distances peut augmenter notablement le rendement du dispositif de l'invention, afin de recevoir au mieux les rayons R aux bons endroits dans les yeux 5. A cet effet, la monture de lunettes peut soit être réglée de manière usuelle par un opticien soit comporter des moyens de réglage connus en soi notamment dans les montures de test utilisées par les oculistes et opticiens et permettant de régler la distance entre lentilles, entre lentilles et yeux, etc.

En variante aux dessins et aux explications ci-dessus, la surface diffractante des lentilles diffractives 19 peut être réduite, notamment en réduisant l'hologramme qui y est réalisé, pour n'occuper que la partie supérieure du verre 13 des dessins, afin d'assurer une vision encore meilleure, à travers la portion inférieure non traitée du verre, au porteur pour de la lecture, etc.

Dans une autre variante, la surface de la lentille 19 elle-même peut être réduite en supprimant sa partie basse pour aussi y dégager la vision du porteur du dispositif de l'invention.

Les dessins ci-joints montrent un cas qui semble favorable à ce jour, dans lequel les sources 3 sont au-dessus de yeux 5, les rayons R sont déviés pour converger dans l'oeil 5 et s'étaler amplement sur une zone déterminée 9 choisie en dessous de la fovéa 21, de façon à ce que cette dernière ne soit pas affectée par ces rayons R mais reste disponible pour pouvoir voir d'autres choses. La présente invention n'est cependant pas limitée à ce cas et comprend toute position et orientation que l'on peut donner aux rayons R, en respectant le fait de disposer les sources 3 en-dehors du champ de vison et en les déviant sur une zone la plus ample possible qui laisse une vision au porteur du dispositif de l'invention.

Un boîtier nécessaire pour l'alimentation et la commande et/ou le réglage de la ou des sources 3 de lumière n'est pas décrit ici du fait qu'il ne participe pas à l'unité d'invention en ce qui concerne ses caractéristiques techniques.

Il doit être compris que la ou les sources lumineuses 3 et/ou le condenseur 15 peuvent être fixés directement sur le support 1, ou indirectement notamment lorsque le condenseur 15 est par exemple collé ou fixé autrement au verre 13 ou à la lentille 19 et qu'éventuellement la ou les sources lumineuses 3 sont fixées au condenseur 15.

Le procédé de photothérapie, qui ne fait pas partie de l'invention, est donc actif au niveau des yeux 5 par des rayons lumineux R d'au moins une longueur d'onde déterminée, émis par au moins une source 3 fixe par rapport à la tête de l'individu à traiter. Ce procédé consiste en fait à disposer la source lumineuses 3 à l'extrême périphérie susdite du champ de vision pour les activités usuelles de cet individu, et à dévier les rayons lumineux R susdits sur une zone déterminée 9 de la rétine 11, choisie de manière à ce que l'individu conserve une vision.

Il doit être entendu que l'invention n'est nullement limitée aux formes de réalisation décrites et que bien des modifications peuvent être apportées à ces dernières sans sortir du cadre des revendications.

Ainsi, on choisit avantageusement la zone déterminée 9 susdite recevant les rayons déviés, de manière à exclure la fovéa 21 quelle que soit la direction du regard dans une plage en dessous du plan passant par l'axe optique X-X des lentilles 19. Ainsi la fovéa 21, région de la rétine 11 qui permet la vision fine, n'est pas éclairée par les rayons R déviés et reste donc disponible pour toute une série d'activités qui ne font pas appel à la vision périphérique (comme la lecture, un travail sur écran, des déplacements dans un environnement sécurisé, ...).

Pour optimiser ledit procédé, on fait converger, dans l'oeil 5, les rayons lumineux R déviés en un lieu 23 (image réelle de la source) situé légèrement en arrière de la pupille 20 de l'oeil 5.

Par ce choix, quel que soit l'angle d'inclinaison de l'oeil 5 dans la plage déterminée ci-dessus, la concentration du flux se produit sur la même région de la rétine 11 (moitié inférieure dans le cas d'exemple représenté). C'est une conséquence de la convergence des rayons et de la position relative de la cornée et du foyer de la lentille 19. Concrètement, cela signifie que la source « apparente » de lumière se déplace avec le regard. Si l'individu baisse les yeux, c'est la partie, inférieure du réseau diffractif de la lentille 19 qui fait la déviation. Lorsque l'individu regarde l'horizon, c'est la partie supérieure de la lentilles 19 qui intervient.

Les rayons lumineux R (figure 2) émis par la source 3 s'étalent en R1 de manière guidée dans le condenseur 15 pour former un faisceau rasant en R2, orienté vers la lentille diffractive 19. Celle-ci réoriente les rayons lumineux R en un faisceau R3 qui converge sur la pupille 20 en une zone plus ample que la surface dé cette dernière. La pupille 20 sélectionne une portion de cette zone et laisse passer un faisceau R4 dévié par les lentilles de l'oeil (cornée et cristallin) pour former au-delà de l'image réelle 23 de la source 3 le faisceau R5 qui atteint ladite zone déterminée 9 de la rétine 5.

Le procédé vient d'être décrit dans un cas où l'on dévie les rayons lumineux par diffraction. On peut cependant aussi dévier les rayons lumineux par réfraction sans sortir de la portée des revendications en annexe.

### Légende des figures

- R: rayons lumineux (comprenant R1 à R5)
- X-X: axe optique de lentille
- V-V: axe de vision
- 1: support
- 3: source(s) lumineuse(s)
- 5: oeil/yeux
- 7: moyens de déviation
- 9: zone déterminée de 11
- 11: rétine de 5
- 12: monture de lunettes
- 13: verre(s) de lunettes ou élément(s) semblable(s)
- 15: condenseur
- 17: ensemble monobloc de condenseurs 15
- 19: lentille diffractive
- 20: pupille de 5
- 21: fovéa de 5
- 23: image réelle de la source lumineuse

## Revendications

1. Dispositif pour la mise en oeuvre d'un procédé de photothérapie actif au niveau des yeux d'un individu, ledit dispositif comprenant :
● un support (1) destiné à être immobilisé sur la tête,
● au moins une source lumineuse (3) montée fixe par rapport à la tête sur le support (1) à la périphérie du champ de vision permettant les activités usuelles de l'individu, ladite source lumineuse (3) émettant des rayons lumineux (R) d'au moins une longueur d'onde déterminée,
● des moyens de déviation (7) agencés pour diriger des rayons lumineux (R) dans les yeux (5) sur une zone déterminée (9) de la rétine (11) de manière à conserver une vision ;
ledit dispositif étant **caractérisé en ce que** les moyens de déviation (7) sont constitués par une lentille diffractive(19) pour chaque oeil (5) ;
et **en ce que** chaque lentille diffractive (19) est constituée d'un élément optique diffractif hors-axe.

2. Dispositif suivant la revendication 1, **caractérisé en ce que** le support (1) précité est constitué par une monture de lunettes (12), les moyens de déviation (7) susdits se présentant sous la forme de verres (13) de lunettes.

3. Dispositif suivant l'une quelconque des revendications 1 ou 2, **caractérisé en ce que** le support (1) est constitué, d'une part, d'une monture (12) de lunettes à verres correcteurs et, d'autre part, d'une applique de lunettes, les moyens de déviation (7) susdits se présentant sous la forme de verres de ladite applique, la ou les sources lumineuses (3) étant montées sur cette applique.

4. Dispositif suivant l'une quelconque des revendications 1 à 3, **caractérisé en ce qu'**il comporte pour chaque oeil (5) une ou plusieurs sources lumineuses (3) et des moyens de déviation (7) distincts agencés pour coopérer avec la ou les sources lumineuses (3) de chaque oeil (5).

5. Dispositif suivant la revendication 4, **caractérisé en ce que** chaque source lumineuse (3) comporte une diode électroluminescente.

6. Dispositif suivant l'une quelconque des revendications 4 ou 5, **caractérisé en ce qu'**il comporte, de préférence distinct pour chaque source lumineuse (3), un condenseur (15) agencé pour orienter les rayons lumineux (R) émis par chacune des sources (3) sur les moyens de déviation (7) précités, et associé à la source lumineuse (3) à la périphérie du champ de vision.

7. Dispositif suivant l'une quelconque des revendications 4 ou 5, **caractérisé en ce que** le condenseur (15) de rayons lumineux (R) est agencé pour orienter lesdits rayons (R) sur la face de la lentille (19) correspondante suivant un angle d'incidence, par rapport à l'axe optique (X-X) de cette lentille (19), prévu pour que la distance séparant cette dernière de l'oeil (5) soit telle que l'image réelle (23) de la source lumineuse soit située dans l'oeil (5), légèrement en arrière de la pupille (20) de celui-ci.

8. Dispositif suivant l'une des revendications précédentes, **caractérisé en ce qu'**on choisit une ouverture numérique de la lentille diffractive (19) de l'ordre de 0,7.

## Patentansprüche

1. Vorrichtung zum Umsetzen eines aktiven Phototherapieverfahrens an den Augen einer Person, wobei die Vorrichtung Folgendes umfasst:
- einen Halter (1), der dazu gedacht ist, am Kopf festgelegt zu werden,
- mindestens eine Lichtquelle (3), die im Verhältnis zum Kopf auf dem Halter (1) am Rand des Blickfeldes fest montiert ist, das die üblichen Tätigkeiten der Person ermöglicht, wobei die Lichtquelle (3) Lichtstrahlen (R) mindestens einer bestimmten Wellenlänge emittiert,
- Ablenkmittel (7), die eingerichtet sind, um Lichtstrahlen (R) derart in die Augen (5) auf eine bestimmte Zone (9) der Netzhaut (11) zu richten, dass eine Sicht bewahrt bleibt;
wobei die Vorrichtung **dadurch gekennzeichnet ist, dass** die Ablenkmittel (7) aus einer Beugungslinse (19) für jedes Auge (5) bestehen;
und dass jede Beugungslinse (19) aus einem außerhalb der Achse liegenden optischen Beugungselement besteht.

2. Vorrichtung nach Anspruch 1, **dadurch gekennzeichnet, dass** der erwähnte Halter (1) aus einem Brillengestell (12) besteht, wobei die genannten Ablenkmittel (7) in Form von Brillengläsern (13) vorliegen.

3. Vorrichtung nach einem der Ansprüche 1 oder 2, **dadurch gekennzeichnet, dass** der Halter (1) einerseits aus einem Brillengestell (12) mit Korrekturgläsern und andererseits aus einem Brillenrahmen besteht, wobei die genannten Ablenkmittel (7) als Gläser des Rahmens vorliegen und die Lichtquelle(n) (3) auf diesem Rahmen montiert ist bzw. sind.

4. Vorrichtung nach einem der Ansprüche 1 bis 3, **dadurch gekennzeichnet, dass** sie für jedes Auge (5) eine oder mehrere Lichtquellen (3) und getrennte Ablenkmittel (7) umfasst, die eingerichtet sind, um mit der oder den Lichtquellen (3) jedes Auges (5) zusammenzuwirken.

5. Vorrichtung nach Anspruch 4, **dadurch gekennzeichnet, dass** jede Lichtquelle (3) eine Leuchtdiode umfasst.

6. Vorrichtung nach einem der Ansprüche 4 oder 5, **dadurch gekennzeichnet, dass** sie bevorzugt getrennt für jede Lichtquelle (3) eine Sammellinse (15) umfasst, die eingerichtet ist, um die Lichtquellen (R), die von jeder der Quellen (3) emittiert werden, auf die genannten Ablenkmittel (7) zu orientieren, und die mit der Lichtquelle (3) am Rand des Blickfeldes verknüpft ist.

7. Vorrichtung nach einem der Ansprüche 4 oder 5, **dadurch gekennzeichnet, dass** die Sammellinse (15) der Lichtstrahlen (R) eingerichtet ist, um die Strahlen (R) auf die Seite der entsprechenden Linse (19) zu orientieren, und zwar in einem Einfallswinkel im Verhältnis zur optischen Achse (X-X) dieser Linse (19), der dafür vorgesehen ist, dass der Abstand, der diese vom Auge (5) trennt, derart ist, dass sich das wirkliche Bild (23) der Lichtquelle in dem Auge (5) etwas hinter seiner Pupille (20) befindet.

8. Vorrichtung nach einem der vorhergehenden Ansprüche, **dadurch gekennzeichnet, dass** eine numerische Apertur der Beugungslinse (19) von ungefähr 0,7 gewählt wird.

## Claims

1. Device for implementing a phototherapy method active at the level of the eyes of an individual, said device comprising:
● a support (1) designed to be immobilised on the head,
● at least one light source (3) mounted fixedly, with respect to the head, on the support (1) at the periphery of the field of vision allowing the usual activities of the individual, said light source (3) emitting light rays (R) of at least one determined wavelength,
● deflection means (7) arranged to direct light rays (R) into the eyes (5) onto a determined zone (9) of the retina (11) so as to retain a vision;
said device being **characterised in that** the deflection means (7) comprise a diffractive lens (19) for each eye (5);
and **in that** each diffractive lens (19) comprises an off-axis diffractive optical element.

2. Device according to claim 1, **characterised in that** said support (1) comprises a spectacle frame (12), said deflection means (7) being in the form of spectacle lenses (13).

3. Device according to any one of claims 1 or 2, **characterised in that** the support (1) comprises, on one hand, a spectacle frame (12) with corrective lenses and, on the other hand, a spectacle attachment, said deflection means (7) being in the form of lenses of said attachment, the light source or light sources (3) being mounted on this attachment.

4. Device according to any one of claims 1 to 3, **characterised in that** it comprises, for each eye (5), one or more light sources (3) and separate deflection means (7) arranged so as to cooperate with the light source or light sources (3) of each eye (5).

5. Device according to claim 4, **characterised in that** each light source (3) comprises a light-emitting diode.

6. Device according to any one of claims 4 or 5, **characterised in that** it comprises, preferably separately for each light source (3), a condenser (15) arranged so as to direct the light rays (R) emitted by each of the sources (3) onto said deflection means (7), and associated with the light source (3) at the periphery of the field of vision.

7. Device according to any one of claims 4 or 5, **characterised in that** the condenser (15) for the light rays (R) is arranged so as to direct said rays (R) onto the face of the corresponding lens (19) at an angle of incidence, with respect to the optical axis (X-X) of said lens (19), provided such that the distance separating the latter from the eye (5) is such that the actual image (23) of the light source is located in the eye (5), slightly behind the pupil (20) thereof.

8. Device according to any one of the preceding claims, **characterised in that** an F number of the diffractive lens (19) of around 0.7 is selected.
